# EUROPEAN PATENT APPLICATION

(11) **EP 1 464 350 A1**
(43) Date of publication of application: **06.10.2004**
(21) Application number: 04006207.7
(22) Date of filing: 16.03.2004
(51) Int. Cl.: A61M 1/36, A61M 1/16

(54) **Device and methods for processing blood in extracorporeal circulation**

(30) Priority: 01.04.2003 IT MI20030647
(71) Applicant: DIDECO S.r.l., I-41037 Mirandola (Province of Modena) (IT)
(72) Inventor: Costa Maianti, Edgardo, 41037 Mirandola (Prov. of Modena) (IT); Ghelli, Nicola, 40018 San Pietro in Casale (IT); Panzani, Ivo, 41037 Mirandola (Prov. of Modena) (IT)
(74) Representative: Modiano, Guido, Dr.-Ing.

(57) **Abstract**

A device (1) for processing blood in extracorporeal blood circuit, which comprises, integrated in a single monolithic assembly, a bubble trap (2), a centrifugal pump (4), a heat exchanger (5), an oxygenator (6) and an arterial filter (7).

## Description

The invention relates to a method and device for processing blood in extracorporeal circulation.

During some surgical procedures it is necessary to establish a circuit for extracorporeal circulation of the blood of the patient. Such circuits may comprise devices such as a pump for conveying blood in the circuit, a heat exchanger in which the blood encounters a heat exchange surface that maintains blood temperature at a desired value, an oxygenation device that is meant to transfer oxygen to the blood, and finally a filter, known as an arterial filter, that is connected in the arterial line and is meant to retain any air bubbles that are present in the blood before the blood is returned to the patient.
In the known art, a venous reservoir is usually also included in the extracorporeal circuit and is used to collect the blood from the patient. The circuit may also include a cardiotomy reservoir which is used for containing the blood collected by recovery from the operating field.

All these devices can be present in the extracorporeal circuit as separate components or can be integrated in various combinations such as disclosed in EPA No. 01118731.7 by the same Applicant.

However, operating technique, in its most advanced form, is now capable of minimizing patient bleeding, and this leads to the fact that for various kinds of procedures operators no longer need to have large quantities of blood available. Additionally, it is desirable to minimize the priming volume required for the extracorporeal circuit. A need exists, therefore, for a device or system which can be used to quickly set up and establish extracorporeal circulation of a small quantity of a patient's blood with minimal priming volume and which can process the blood in a safe and effficient manner. An object of the present invention is therefore to provide a device for processing blood in extracorporeal circulation that can be used when the operators do not require the availability of large quantities of blood, ensuring optimum and safe processing of the blood and convenient management by the operators.

This object is achieved by a device for processing blood in extracorporeal circulation, according to the invention that comprises, integrated in a single monolithic assembly, a bubble trap, a centrifugal blood pump, an oxygenator, a heat exchanger and an arterial blood filter, all of which are inter connected by means of various tubing lines and/or ducts. The integrated structure forms a complete system which includes all of the components required in an extracorporeal blood circuit used during heart bypass surgery or used to assist the heart during beating heart surgery. The integrated structure is configured to be shipped to a user as a completely assembled and interconnected system or alternatively, to more efficiently use packaging space some minimal assembly might be required by the user.

The bubble trap has an inlet connected to a venous line for receiving blood from a patient and an outlet connected to supply blood to an inlet of a centrifugal pump. The centrifugal pump has an outlet that is connected at the inlet of a heat exchanger, the outlet of which is connected to the inlet of an oxygenator. The oxygenator has an outlet that is connected to an inlet of an arterial filter. The arterial filter is provided with an outlet connector for coupling to an arterial line for the return of the processed blood to the patient.

In one embodiment the invention comprises an integrated device for oxygenating and filtering blood flowing through an extracorporeal blood circuit. The integrated device comprises a bubble trap having an inlet for receiving venous blood and an outlet for supplying venous blood. The device includes a blood pump having an inlet connected to receive blood from the outlet of the bubble trap and an outlet connected to a blood inlet of a heat exchanger. The heat exchanger has a blood outlet for supplying temperature controlled venous blood to the inlet of an oxygenator. The oxygenator has an outlet for supplying oxygenated blood to the inlet of an arterial blood filter. The arterial blood filter has an outlet adapted for connection to an arterial line for return of oxygenated blood to the patient. The integrated device includes a monolithic housing having a first portion for defining the bubble trap, a second portion for defining the blood pump, a third portion for defining the heat exchanger, a fourth portion for defining the oxygenator and a fifth portion for defining the arterial blood filter. The blood pump may comprise a centrifugal pump that is positioned within the monolithic housing such that an axis of the centrifugal pump is horizontal.

In another embodiment the invention comprises a system for establishing an extracorporeal blood circuit. The system includes a bubble trap, a blood pump, a heat exchanger, an oxygenator, an arterial blood filter and a housing for incorporating and interconnecting the bubble trap, the blood pump, the heat exchanger, the oxygenator, and the arterial blood filter into a monolithic structure. The housing has an inlet for receiving venous blood from a patient and supplying the venous blood to the bubble trap and an outlet for supplying oxygenated blood from the arterial blood filter to a patient. The blood pump may comprise a centrifugal pump which is positioned within the housing such that an axis of the pump is horizontal.

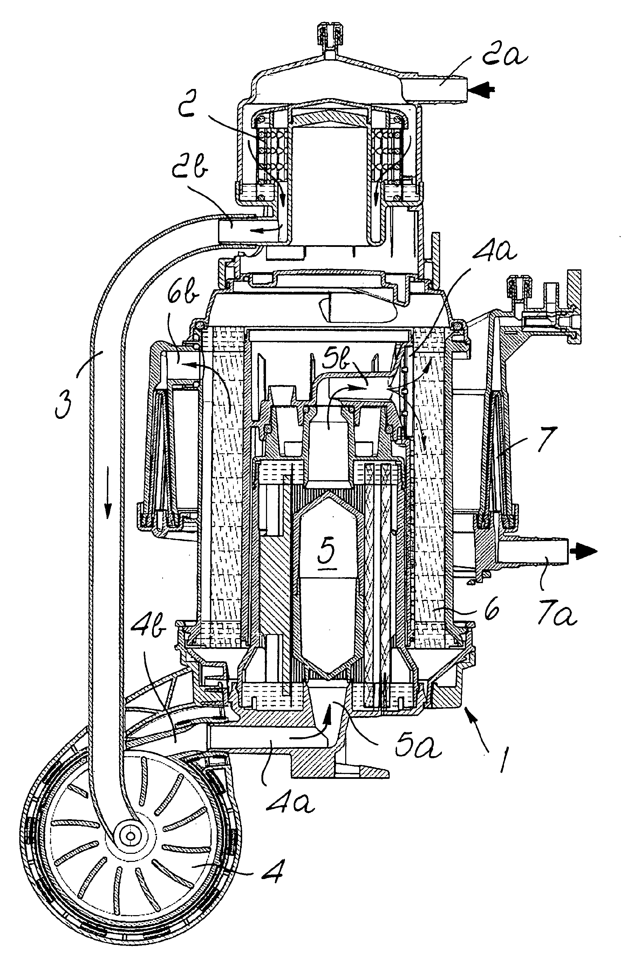

Further characteristics and advantages will become better apparent from the description of a preferred but not exclusive embodiment of the invention, illustrated by way of nonlimiting example in the accompanying drawings. Figure 1 is a longitudinal sectional view of the invention.

With reference to Figure 1, the reference numeral 1 generally designates the device according to the invention, which comprises, integrated in a single monolithic assembly, a series of components that are interconnected by various tubing lines and/or ducts to form a complete extracorporeal blood circuit which can be used to process a patient's blood during heart bypass surgery or beating heart surgery.

The device includes a bubble trap 2. Bubble trap 2 is provided with an inlet connector 2a for connection to a venous line for receiving blood from the patient.

An outlet connector 2b of bubble trap 2 is connected at one end of duct 3. The other end of duct 3 is connected to an inlet of centrifugal pump 4. Centrifugal pump 4 has a transparent enclosure which defines an outlet connector 4b which is connected to delivery duct 4a, connected to provide blood from centrifugal pump 4 to an inlet connector 5a of heat exchanger 5. Heat exchanger 5 is provided with an outlet connector 5b that is configured to convey the blood to inlet 6a of oxygenator 6.

From the outlet 6b of the oxygenator 6, the blood reaches arterial filter 7, which is provided with an outlet connector 7a for connection to an arterial line for the return of the blood to the patient.

The transparent enclosure of the centrifugal pump 4 is connected monolithically to the end face of the structure that comprises the heat exchanger 5 and the oxygenator 6. Centrifugal pump 4 is oriented so that the pump axis is horizontal and outlet connector 4b is positioned at the top of the pump. This arrangement is beneficial for several reasons. First, it essentially ensures the complete removal of any air contained in the pump during its filling since any air bubbles in the device will rise because of their buoyancy and be expelled through the outlet connector at the top of the pump. Second, since the axis of the centrifugal pump is horizontal the entire rotor and pump chamber are visible to the operator through the transparent enclosure. This allows the operator to visually locate any air bubbles which may pass into or through the pump and to take appropriate corrective action in response thereto.

The device according to the invention is highly functional. The choice to adopt as a component of the assembly a centrifugal pump to produce blood circulation in the extracorporeal circuit ensures that the partial vacuum of the blood that flows in intake through the venous line and originates from the patient is limited to values that do not cause deterioration of the vessel of the patient from which the blood is drawn, as might instead occur with a volumetric pump.

It is desirable to prevent air bubbles from reaching the centrifugal pump since they could undergo, during passage through the pump, a micronization that could prevent the arterial filter 7 from capturing them. The bubble trap 2 which is connected upstream from the pump in the circuit is provided to perform this function.

The device of the present invention may be used as a fast, efficient and convenient alterative to the use of various individual components in a traditional extracorporeal blood circuit. Since the structure of the device incorporates these various individual components into a preconnected monolithic structure it can be set up and ready for use very quickly. Further, the present invention is advantageous for use in beating heart surgery for several reasons. First, since part of the blood is diverted into an extracorporeal circuit the heart has less blood to pump and therefore beats less making it easier for the surgeon to work on the heart. Second, in cases where it is necessary to change to full bypass during the surgery the monolithic device of the present invention includes an oxygenator and other components which provide the capacity and function necessary to accommodate the change. The present invention also includes methods of using the device to process blood during heart bypass or beating heart surgery.

It should be understood that the embodiments disclosed herein represent presently preferred embodiments of the invention. Various modifications and additions may be made to these embodiments without departing from the spirit and scope of the invention.

The disclosures in Italian Patent Application No. MI2003A000647 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An integrated device for oxygenating and filtering blood flowing through an extracorporeal blood circuit **characterized in that** it comprises:
a bubble trap having an inlet for receiving venous blood and an outlet for supplying venous blood;
a blood pump having an inlet connected to receive blood from the outlet of the blood reservoir and an outlet;
a heat exchanger having a blood inlet connected to receive venous blood from the outlet of the pump and a blood outlet for supplying temperature controlled venous blood;
an oxygenator having an inlet connected to receive venous blood from the outlet of the heat exchanger and an outlet for supplying oxygenated blood;
an arterial blood filter having an inlet connected to receive oxygenated blood from the outlet of the oxygenator and an outlet for supplying filtered oxygenated blood; and
a monolithic housing including a first portion for defining the bubble trap, a second portion for defining the blood pump, a third portion for defining the heat exchanger, a fourth portion for defining the oxygenator and a fifth portion for defining the arterial blood filter.

2. The integrated device of claim 1, **characterized in that** the blood pump comprises a centrifugal pump.

3. The integrated device of claim 2, **characterized in that** the centrifugal pump has an axis and wherein the centrifugal pump is positioned within the monolithic housing such that the axis of the centrifugal pump is horizontal.

4. A system for establishing an extracorporeal blood circuit **characterized in that** it comprises:
a bubble trap;
a blood pump;
a heat exchanger;
an oxygenator;
an arterial blood filter; and
a housing for incorporating and interconnecting the bubble trap, the blood pump, the heat exchanger, the oxygenator and the arterial blood filter into a monolithic structure, the housing having an inlet for supplying venous blood to the blood reservoir and an outlet for supplying oxygenated blood from the arterial blood filter.

5. The system of claim 4, **characterized in that** the blood pump comprises a centrifugal pump.

6. The system of claim 5, **characterized in that** the centrifugal pump has an axis and wherein the centrifugal pump is positioned within the housing such that the axis of the centrifugal pump is horizontal.
